# EUROPEAN PATENT SPECIFICATION

(11) **EP 4 263 490 B1**
(45) Date of publication and mention of the grant of the patent: **04.06.2025**
(21) Application number: 21824603.1
(22) Date of filing: 16.12.2021
(51) Int. Cl.: C07C 227/42, C07C 229/52

(54) **METHOD FOR OBTAINING CRYSTALLINE DIETHYLAMINO HYDROXYBENZOYL HEXYL BENZOATE**
VERFAHREN ZUR HERSTELLUNG VON KRISTALLINEM DIETHYLAMINOHYDROXYBENZOYLHEXYLBENZOAT
PROCÉDÉ D'OBTENTION DE DIÉTHYLAMINO HYDROXYBENZOYL HEXYL BENZOATE CRISTALLIN

(30) Priority: 18.12.2020 EP 20215330
(43) Date of publication of application: 25.10.2023
(73) Proprietor: BASF SE, 67056 Ludwigshafen am Rhein (DE)
(72) Inventor: KRONEMAYER, Helmut, 67056 Ludwigshafen (DE); WLOCH, Sebastian, 67056 Ludwigshafen (DE); MELZER, Andreas, 67056 Ludwigshafen (DE); BLANCHOT, Mathieu, 67056 Ludwigshafen (DE); SCHEIN-ALBRECHT, Karin, 67056 Ludwigshafen (DE); KAUFHOLD, Dennis, 67056 Ludwigshafen (DE); EHLIS, Thomas, 4133 Schweizerhalle (Muttenz) (CH)
(74) Representative: BASF IP Association
(86) International application number: PCT/EP2021/086103
(87) International publication number: WO 2022/129284

(56) References cited:
- US-A1- 2005 165 099
- US-A1- 2014 349 115
- DATABASE WPI Week 2020, Derwent World Patents Index; AN 2020-04803J, XP002803030

## Description

### Field of invention

The presently claimed invention relates to a method for obtaining crystalline diethylamino hydroxybenzoyl hexyl benzoate. Further, the presently claimed invention relates to crystalline diethylamino hydroxybenzoyl hexyl benzoate obtained by the method and to a cosmetic composition comprising crystalline diethylamino hydroxybenzoyl hexyl benzoate.

### Background of the invention

UV radiation causes harmful effects on the human skin such as sunburn, phototoxic and photo allergenic reactions, acceleration of skin aging and increase in the risk of skin cancer. To protect the human skin from UV radiation, sunscreen or cosmetic compositions comprising UV filters (also referred to as UV absorbers) are used.

In general, UV light can be divided into UV-A radiation (320 - 400 nm) and UV-B radiation (280 - 320 nm). Since 2006, the EU commission has recommended that all sunscreen or cosmetic compositions should have an UV-A protection factor, which is at least one third of the labelled sun protection factor (SPF), wherein the sun protection factor refers mainly to the UV-B protection. 2-(4'-Diethylamino-2'-hydroxybenzoyl)benzoic acid hexyl ester is an effective UV-A filter. It is represented by formula (I).

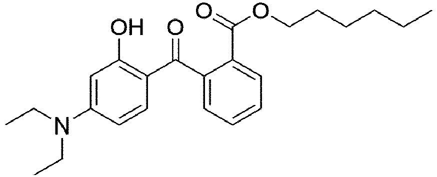

2-(4'-Diethylamino-2'-hydroxybenzoyl)benzoic acid hexyl ester (I) preparation is known, e.g. from DE 10011317, EP2155660, and WO 2003097578.

However, the product obtained from known processes are associated with several disadvantages. US 2005/165099 discloses the isolation of a crystalline form of n-hexylyl 2-(4-N,N-diethylamino-2-hydroxybenzoyl)benzoate.

Generally, 2-(4'-diethylamino-2'-hydroxybenzoyl)benzoic acid hexyl ester (I) is obtained as a melt after the completion of synthesis process and it stored in the same form. Crystal growth occurs in the melt after about six weeks at room temperature. At the time of use, the user is required to heat the entire pack to a temperature above the melting point of (I) to be able to remove product from the storage containers.

The product contains phthalic acid dihexyl ester (II) (also known as PSDHE, dihexylphthalate) as an impurity. The PSDHE content should be as low as possible, since this substance is toxic; it can damage fertility and may have harmful effects on the unborn child. According to the harmonized classification and labelling (ATP05), the allowable amount of PSDHE (II) in the final product is up to 150 ppm.

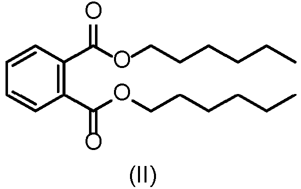

Further, the product obtained by known processes contains rhodamine B type dyes (e.g. [9-(2-carboxyphenyl)-6-diethylamino-3-xanthenylidene]-diethylammonium salts) (III) and corresponding esters (IV) as impurities, which lead to an undesired discoloration of the final product.

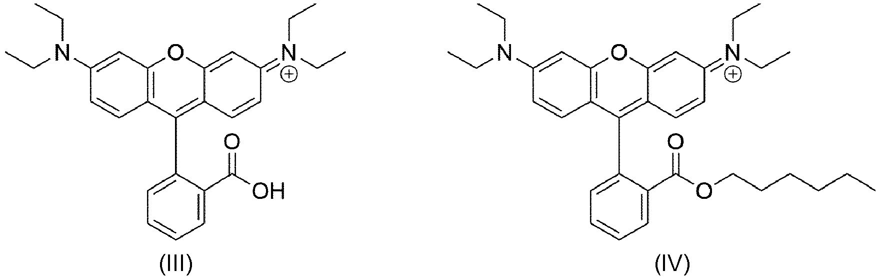

Due to these challenges, there is an ongoing need for a fast and efficient method for obtaining crystalline 2-(4'-diethylamino-2'-hydroxybenzoyl)benzoic acid hexyl ester (I) as well as obtaining crystalline (I) having a low amount of (II), and low amounts of (III) and (IV).

Accordingly, it is an object of the present invention to provide a method for obtaining crystalline 2-(4'-diethylamino-2'-hydroxybenzoyl)benzoic acid hexyl ester (I). Further, it is an object to provide a method to obtain crystalline 2-(4'-diethylamino-2'-hydroxybenzoyl)benzoic acid hexyl ester (I) having a low amount of phthalic acid dihexyl ester (II). Furthermore, it is desired that the method provides crystalline 2-(4'-diethylamino-2'-hydroxybenzoyl)benzoic acid hexyl ester having low amounts of rhodamine based impurities (III) and (IV).

### Summary of the invention

Surprisingly, it is found that at least one of the above objects is achieved by the method according to the presently claimed invention. The method of the presently claimed invention provides crystalline 2-(4'-diethylamino-2'-hydroxybenzoyl)benzoic acid hexyl ester (I) having a low PSDHE (II) content (in the range of 1 to 250 ppm), a low rhodamine (III) content (the range of 1 to 100 ppm) and a low rhodamine hexyl ester (IV) content (the range of 1 to 120 ppm).

Thus, an aspect of the presently claimed invention directed to a method for obtaining crystalline diethylamino hydroxybenzoyl hexyl benzoate (I); the method comprises steps (a) to (d),
(a) providing a feed comprising diethylamino hydroxybenzoyl hexyl benzoate (I) and at least one crystallization solvent, and heating the feed to obtain a solution,
   wherein the diethylamino hydroxybenzoyl hexyl benzoate (I) has
      i) a phthalic acid dihexyl ester (PSDHE) (II) content in the range of 5000 to 30000 ppm;
      ii) a rhodamine (III) content in the range of 500 to 10000 ppm; and
      iii) a rhodamine hexyl ester (IV) content in the range of 500 to 10000 ppm; each based on the weight of diethylamino hydroxybenzoyl hexyl benzoate (I); and
   wherein the amount of the diethylamino hydroxybenzoyl hexyl benzoate (I) in the feed is in the range of 15.0 and 65.0 wt% based on the total weight of the feed,
(b) cooling the solution of step (a) to a temperature such that the ratio of c:c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
   wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by using the method steps (b) to (d).

In a preferred embodiment, the method for obtaining crystalline diethylamino hydroxybenzoyl hexyl benzoate (I) comprises steps (a) to (d),
(a) providing a feed comprising diethylamino hydroxybenzoyl hexyl benzoate (I) and at least one crystallization solvent, and heating the feed to obtain a solution,
   wherein the diethylamino hydroxybenzoyl hexyl benzoate (I) has
      i) a phthalic acid dihexyl ester (PSDHE) (II) content in the range of 5000 to 30000 ppm;
      ii) a rhodamine (III) content in the range of 500 to 10000 ppm; and
      iii) a rhodamine hexyl ester (IV) content in the range of 500 to 10000 ppm; each based on the weight of diethylamino hydroxybenzoyl hexyl benzoate (I); and
   wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
   wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by using the method steps (b) to (d).

Another aspect of the presently claimed invention is directed to crystalline diethylamino hydroxybenzoyl hexyl benzoate (I) obtained by the method described above.

Another aspect of the presently claimed invention is directed to the crystalline diethylamino hydroxybenzoyl hexyl benzoate (I) obtained by the method of the presently claimed invention having a phthalic acid dihexyl ester (PSDHE) content in the range of 1 to 250 ppm, based on the weight of the crystalline diethylamino hydroxybenzoyl hexyl benzoate (I).

The crystalline diethylamino hydroxybenzoyl hexyl benzoate (I) has
i) a phthalic acid dihexyl ester (PSDHE) content in the range of 1 to 250 ppm;
ii) a rhodamine content in the range of 1 to 100 ppm; and
iii) a rhodamine hexyl ester content in the range of 1 to 120 ppm;
each based on the weight of the crystalline diethylamino hydroxybenzoyl hexyl benzoate (I).

### Brief Description of the drawings

The disclosure described herein is illustrated by way of example and not by way of limitation in the accompanying figures.
**Figure 1** is a graph related to the equilibrium solubility of (I) in 1-hexanol at different temperatures.
**Figure 2** is a high-resolution photograph of the crystalline (I) obtained by the process of comparative example 1.
**Figure 3** is a high-resolution photograph of the crystalline (I) obtained by the process of the present invention (example 1).

### Detailed Description

Before the present compositions and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

Surprisingly, it is found that the method according to the presently claimed invention provides crystalline 2-(4'-diethylamino-2'-hydroxybenzoyl)benzoic acid hexyl ester (I) having a low PSDHE (II) content as well as low content of rhodamine based impurities (III) and (IV). The method of the presently claimed invention is fast and efficient.

The method of the presently claimed invention improves the quality and purity of crystalline (I) and reduces the time required for the crystallization by selection of appropriate crystallization process parameters such as crystallization solvent, degree of supersaturation, the amount of seed crystals of (I), the temperature of seeding and the temperature as well as time of each process step.

Accordingly, an aspect of the presently claimed invention is directed to a method for obtaining crystalline diethylamino hydroxybenzoyl hexyl benzoate (I) comprising steps (a) to (d),
(a) providing a feed comprising diethylamino hydroxybenzoyl hexyl benzoate (I) and at least one crystallization solvent, and heating the feed to obtain a solution,
   wherein the diethylamino hydroxybenzoyl hexyl benzoate (I) has
      i) a phthalic acid dihexyl ester (PSDHE) (II) content in the range of 5000 to 30000 ppm;
      ii) a rhodamine (III) content in the range of 500 to 10000 ppm; and
      iii) a rhodamine hexyl ester (IV) content in the range of 500 to 10000 ppm; each based on the weight of diethylamino hydroxybenzoyl hexyl benzoate (I); and
   wherein the amount of the diethylamino hydroxybenzoyl hexyl benzoate (I) in the feed is in the range of 15.0 and 65.0 wt% based on the total weight of the feed,
(b) cooling the solution of step (a) to a temperature such that the ratio of c:c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
   wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by using the method steps (b) to (d).

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30, the UVA protection factor has to be at least 10.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

In a preferred embodiment, the method for obtaining crystalline diethylamino hydroxybenzoyl hexyl benzoate (I) comprises steps (a) to (d),
(a) providing a feed comprising diethylamino hydroxybenzoyl hexyl benzoate (I) and at least one crystallization solvent, and heating the feed to obtain a solution,
   wherein the diethylamino hydroxybenzoyl hexyl benzoate (I) has
      i) a phthalic acid dihexyl ester (PSDHE) (II) content in the range of 5000 to 30000 ppm;
      ii) a rhodamine (III) content in the range of 500 to 10000 ppm; and
      iii) a rhodamine hexyl ester (IV) content in the range of 500 to 10000 ppm; each based on the weight of diethylamino hydroxybenzoyl hexyl benzoate (I); and
   wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
   wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by using the method steps (b) to (d).

In a preferred embodiment, the method for obtaining crystalline diethylamino hydroxybenzoyl hexyl benzoate (I) involves the recrystallization step. Thus, the process comprises steps (a) to (e),
(a) providing a feed comprising diethylamino hydroxybenzoyl hexyl benzoate (I) and at least one crystallization solvent, and heating the feed to obtain a solution,
   wherein the diethylamino hydroxybenzoyl hexyl benzoate (I) has
      i) a phthalic acid dihexyl ester (PSDHE) (II) content in the range of 5000 to 30000 ppm;
      ii) a rhodamine content (III) in the range of 500 to 10000 ppm; and
      iii) a rhodamine hexyl ester (IV) content in the range of 500 to 10000 ppm; each based on the weight of diethylamino hydroxybenzoyl hexyl benzoate (I); and
   wherein the amount of the diethylamino hydroxybenzoyl hexyl benzoate (I) in the feed is in the range of 15.0 and 65.0 wt% based on the total weight of the feed,
(b) cooling the solution of step (a) to a temperature such that the ratio of c:c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
   wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by using the method steps (b) to (d).

In a preferred embodiment, the crystallization solvent is at least one selected from the group consisting of C₄-C₈ alcohols.

In a preferred embodiment, the crystallization solvent is 1-hexanol.

In general, the last step of the synthesis of diethylamino hydroxybenzoyl hexyl benzoate (I) is esterification of diethylamino hydroxybenzoyl benzoic acid with 1-hexanol. The product obtained from the esterification step comprises a mixture of diethylamino hydroxybenzoyl hexyl benzoate (I) and 1-hexanol.

In a preferred embodiment, the product obtained from the esterification comprising diethylamino hydroxybenzoyl hexyl benzoate (I) and 1-hexanol is directly used as a feed for the crystallization.

In a preferred embodiment, the amount of (I) in the feed is in the range of 15.0 and 65.0 wt%, more preferably in the range of 25.0 to 60.0 wt%, even more preferably in the range of 35.0 to 50.0 wt%, most preferably in the range of 40.0 to 50.0 wt%, and in particular preferably in the range of 45.0 to 49.0 wt% based on the total weight of the feed.

In a preferred embodiment, the amount of 1-hexanol in the feed is in the range of 35.0 to 85.0 wt%, more preferably in the range of 40.0 to 75.0 wt%, most preferably in the range of 50.0 to 65.0 %, and in particular preferably in the range from 50.0 to 60.0 wt%, based on the total weight of the feed.

The feed also contains impurities.

In a preferred embodiment, the amount of phthalic acid dihexyl ester (PSDHE) (II) in the feed is in the range of 8000 to 20000 ppm and more preferably in the range of 12000 to 18000 ppm based on the weight of diethylamino hydroxybenzoyl hexyl benzoate (I).

In a preferred embodiment, the amount of rhodamine (III) in the feed is in the range of 500 to 6000 ppm and more preferably in the range of 1000 to 4000 ppm based on the weight of diethylamino hydroxybenzoyl hexyl benzoate (I).

In a preferred embodiment, the amount of rhodamine hexyl ester (IV) in the range of 1000 to 10000 ppm and more preferably in the range of 5000 to 10000 ppm based on the weight of diethylamino hydroxybenzoyl hexyl benzoate (I).

In a preferred embodiment, an additional amount of 1-hexanol is mixed with the product obtained from the esterification comprising diethylamino hydroxybenzoyl hexyl benzoate (I) and 1-hexanol to obtain feed. The amount of 1-hexanol required depends upon the concentration of (I) to be achieved in the feed in step (a).

In a preferred embodiment, in step (a) the feed is heated to a temperature in the range of 25 to 80 °C. In a more preferred embodiment, in step (a) the feed is heated to a temperature in the range of 35 to 80 °C, most preferably in the range of 35 to 60 °C and in particular preferably in the range of 38 to 60 °C.

In a preferred embodiment, in step (a) the feed is heated to 48°C.

In a preferred embodiment, in step (a) the feed is heated to 49°C.

In a preferred embodiment, in step (b) the solution is cooled to a temperature in the range of by 18 to 35 °C, more preferably in the range of 18 to 30 °C and most preferably in the range of 18 to 28 °C.

In a preferred embodiment, in step (b) the solution is cooled to 25°C.

In a preferred embodiment, in step (b) the solution is cooled to 26°C.

### Degree of supersaturation

The degree of supersaturation (c:c*) is an important aspect of the crystallization process of the presently claimed invention.

In a preferred embodiment, the ratio of c:c* is in the range from 1.1:1.0 to 9.0:1.0, more preferably in the range of 1.5:1.0 to 7.0:1.0 and most preferably in the range from 2.0:1.0 to 6.0:1.0.

In a preferred embodiment, the ratio of c:c* is 3.8:1.0.

In a preferred embodiment, the ratio of c:c* is 4.0:1.0.

In a preferred embodiment, the ratio of c:c* is 4.1:1.0.

### Seeding

The crystallization of (I) on its own is very slow due to the high metastable character of liquid (I). Seeding of the supersaturated solution is an important step during the crystallization of the presently claimed invention. Due to seeding, the crystallization starts early as compared to that without seed crystals. Hence, the overall process of the presently claimed invention provides crystalline (I) faster as compared to a process that does not involve the step of seeding.

In addition to the other process parameters, carrying out the seeding at a right temperature and using a right amount of crystals of (I) followed by the stirring at the temperature of seeding are important for the success of the presently claimed invention.

The quality of crystalline (I) obtained during the crystallization depends upon the temperature at which seeding is carried out as well as upon the amount of seed crystals.

In a preferred embodiment, in step (c) the seeding is carried out at a temperature in the range of 18 to 35 °C, more preferably in the range of 18 to 30 °C and most preferably in the range of 18 to 28 °C.

In a preferred embodiment, in step (c) the seeding is carried out at 25°C.

In a preferred embodiment, the seed crystals of diethylamino hydroxybenzoyl hexyl benzoate (I) are employed in step (c) in at least one form selected from a solid crystalline mass and a suspension in a solvent.

In a preferred embodiment, the seed crystals of diethylamino hydroxybenzoyl hexyl benzoate (I) are employed in step (c) in the form of a suspension in the crystallization solvent in step (a).

In a more preferred embodiment, the seed crystals of diethylamino hydroxybenzoyl hexyl benzoate (I) are employed in step (c) in the form of a suspension in 1-hexanol.

In a preferred embodiment, the amount of the seed crystals is in the range of 0.1 to 15.0 wt%, more preferably in the range of 0.5 to 10.0 wt%, even more preferably in the range of 1.0 to 5.0 wt% and most preferably in the range of 1.5 to 4.5 wt%, based on the weight of diethylamino hydroxybenzoyl hexyl benzoate (I).

In a preferred embodiment, the amount of the seed crystals is 1.5 wt% based on the weight of diethylamino hydroxybenzoyl hexyl benzoate (I) in the solution.

In a preferred embodiment, the amount of the seed crystals is 3.6 wt% based on the weight of diethylamino hydroxybenzoyl hexyl benzoate (I) in the solution.

In a preferred embodiment, after seeding, the supersaturated solution comprising seed crystals is agitated for 1 to 8 hours, more preferably for 2 to 6 hours and most preferably for 3 to 4 hours.

In a preferred embodiment, step (d) involves cooling the solution obtained in step (c) -10 to 15 °C; more preferably to -5 to 10 °C and most preferably to 0 to 5 °C.

In a preferred embodiment, in step (d) the solution is cooled to 0°C.

In a preferred embodiment, step (d) involves cooling the solution obtained in step (c) at a cooling rate of 1 to 15 °C/h, more preferably at 3 to 10 °C/h and most preferably at 3 to 8 °C/h.

In a preferred embodiment, in step (d) the solution is cooled at a cooling rate of 5 °C/h.

In a preferred embodiment, in step (d) the solution is cooled in a linear manner.

In a preferred embodiment, in step (d) the solution is cooled with a piecewise linear cooling profile.

In a preferred embodiment, in step (d) the solution is cooled with a parabolic cooling profile with increasing cooling rates.

In a preferred embodiment, in step (d) the solution is cooled with a dynamic cooling profile to maintain a relatively constant supersaturation in the crystallizer.

In a preferred embodiment, in step (d) the solution is cooled with vacuum cooling method.

In a preferred embodiment, in step (d) the separation of crystals is carried out by filtration while maintaining the temperature of the solution within ± 5 °C of the temperature to which the solution is cooled in step (d).

In a preferred embodiment, step (d) further comprises a step of washing the separated crystalline diethylamino hydroxybenzoyl hexyl benzoate (I) with a washing solvent.

In a preferred embodiment, the washing of the separated crystalline diethylamino hydroxybenzoyl hexyl benzoate (I) is carried out with a washing solvent having a temperature within ± 5 °C of the temperature to which the solution is cooled in step (d).

In a preferred embodiment, the washing solvent is at least one selected from the crystallization solvent used in step (a), the feed and a saturated solution of (I) in the solvent used in step (a).

In a preferred embodiment, the washing solvent is the crystallization solvent used in step (a).

In a preferred embodiment, the washing solvent is cooled to a temperature in the range of -5 to 5 °C.

In a preferred embodiment, the method of the presently claimed invention further comprises the following steps,
i. dissolving the crystalline diethylamino hydroxybenzoyl hexyl benzoate (I) obtained in step (d) or step (e) in a purification solvent selected from toluene and cyclohexane;
ii. contacting the solution obtained in step (i) with an adsorbent selected from silica and charcoal; and
iii. removal of solvent from the solution obtained in step (ii) to obtain crystalline diethylamino hydroxybenzoyl hexyl benzoate (I).

In a preferred embodiment, the crystallization is carried out in a crystallizer selected from a stirred vessel with baffles, force circulation crystallizer, draft tube crystallizer, draft tube baffled crystallizer, and an Oslo type crystallizer.

The method of the presently claimed invention provides crystalline diethylamino hydroxybenzoyl hexyl benzoate (I) with a yield of 85.0 to 99.5 %, more preferably 90.0 to 99.0 % based on the total of (I) in the feed.

The crystalline diethylamino hydroxybenzoyl hexyl benzoate (I) obtained by the method of the presently claimed invention has a phthalic acid dihexyl ester (PSDHE) (II) content in the range of 1 to 250 ppm, preferably in the range of 1 to 200 ppm, more preferably in the range of 1 to 150 ppm, even more preferably in the range of 1 to 100 ppm, even more preferably in the range of 1 to 50 ppm, most preferably in the range of 1 to 25 ppm and in particular preferably in the range of 1 to 10 ppm based on the weight of the crystalline diethylamino hydroxybenzoyl hexyl benzoate (I).

The method of the presently claimed invention provides crystalline diethylamino hydroxybenzoyl hexyl benzoate (I) having
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm;
each based on the weight of the crystalline diethylamino hydroxybenzoyl hexyl benzoate (I).

The crystalline diethylamino hydroxybenzoyl hexyl benzoate (I) obtained by the method of the presently claimed invention has a phthalic acid dihexyl ester (PSDHE) (II) content in the range of 1 to 250 ppm, preferably in the range of 1 to 200 ppm, more preferably in the range of 1 to 150 ppm, even more preferably in the range of 1 to 100 ppm, even more preferably in the range of 1 to 50 ppm, most preferably in the range of 1 to 25 ppm and in particular preferably in the range of 1 to 10 ppm based on the weight of the crystalline diethylamino hydroxybenzoyl hexyl benzoate (I).

The crystalline diethylamino hydroxybenzoyl hexyl benzoate (I) obtained has a rhodamine (III) content in the range of 1 to 100 ppm, more preferably in the range of 1 to 80 ppm, even more preferably in the range of 1 to 50 ppm, most preferably in the range of 1 to 25 ppm and in particular preferably in the range of 1 to 10 ppm based on the weight of the crystalline diethylamino hydroxybenzoyl hexyl benzoate (I).

The crystalline diethylamino hydroxybenzoyl hexyl benzoate (I) obtained has a rhodamine hexyl ester (IV) content in the range of 1 to 120 ppm, more preferably in the range of 1 to 100 ppm, even more preferably in the range of 1 to 80 ppm, even more preferably in the range of 1 to 50 ppm, most preferably in the range of 1 to 25 ppm and in particular preferably in the range of 1 to 10 ppm based on the weight of the crystalline diethylamino hydroxybenzoyl hexyl benzoate (I).

The process of the presently claimed invention provides crystalline material having a narrow size distribution. The crystals are uniform, and the crystalline mass obtained has a low amount of fines.

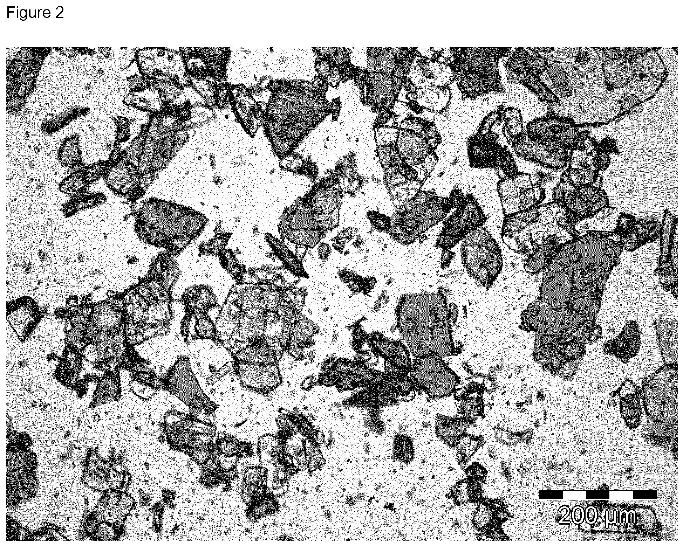

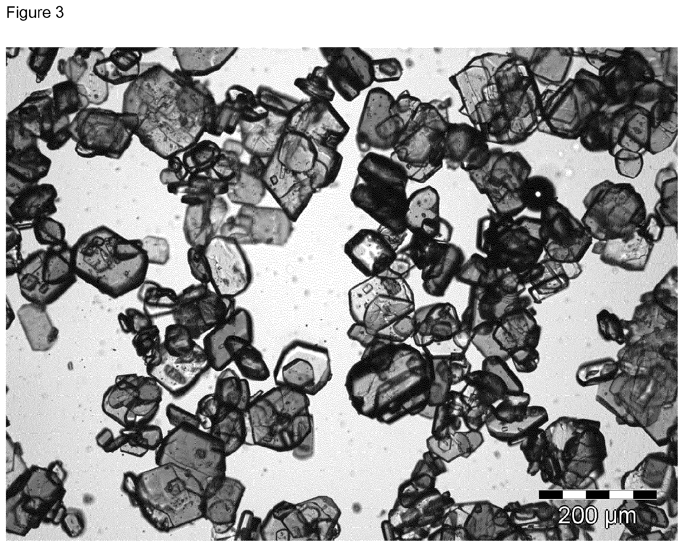

High resolution photographs of the crystalline mass of (I) obtained with the process of WO 03097578 and that obtained by the process of the presently claimed invention are shown in **Figure 2** and **Figure 3** respectively. The crystalline mass obtained by the process of the presently claimed invention shows uniform crystals and a low amount of fines.

A uniform crystalline mass is desired since it provides various advantages such as ease of formulation and better performance of cosmetic composition comprising the product.

Due to the low amount of fines, crystalline (I) can be separated from the mother liquor at a high filtration rate with lower incidence of filter clogging during manufacturing.

Advantageously, an early start of crystallization on account of seeding and a faster filtration due to lower amount of fines results in a reduction in batch time for the process. Such reduction in batch time translate in significant economic advantage in industry.

Another aspect of the presently claimed invention is directed to crystalline diethylamino hydroxybenzoyl hexyl benzoate (I) obtained by the method as described above, having
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm;
each based on the weight of the crystalline diethylamino hydroxybenzoyl hexyl benzoate (I).

Another aspect of the presently claimed invention is directed to a cosmetic composition comprising crystalline diethylamino hydroxybenzoyl hexyl benzoate (I) and a carrier, wherein the crystalline diethylamino hydroxybenzoyl hexyl benzoate (I) has
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm,
each based on the weight of crystalline diethylamino hydroxybenzoyl hexyl benzoate (I).

In a preferred embodiment, the cosmetic composition further comprises additives and adjuvants.

In a preferred embodiment, the cosmetic composition can be in the form of creams, gels, lotions, alcoholic and aqueous/alcoholic solutions, emulsions, wax/fat compositions, stick preparations, powders or ointments.

In a preferred embodiment, the cosmetic composition further comprises adjuvants and additives selected from mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, antioxidants, hydrotropic agents, preservatives, insect repellents, self-tanning agents, solubilizers, perfume oils, colorants, bacteria-inhibiting agents and the like.

In a preferred embodiment, the cosmetic compositions according to the presently claimed invention may further comprise as adjuvants, anti-foams, structurants, solubilizers, opacifiers, complexing agents, propellants, coupler and developer components as oxidation dye precursors, reducing agents and oxidizing agents.

In a preferred embodiment, the cosmetic compositions are contained in a wide variety of cosmetic preparations, especially the following preparations:
- skin-care preparations, e.g. skin-washing and cleansing preparations in the form of tablet-form or liquid soaps, synthetic detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eye shadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, parfume), parfume oils or parfume creams;
- cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hair-sprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile.

Yet another aspect of the presently claimed invention is directed to the use of the crystalline 2-(4'-diethylamino-2'-hydroxybenzoyl)benzoic acid hexyl ester (I) obtained according to the claimed process as UV filter in a cosmetic composition.

The presently claimed invention offers one or more of the following advantages:
1) The method of the presently claimed invention provides crystalline (I) having a high purity and a low amount of impurities (II), (III) and (IV). The method of the presently claimed invention provides crystalline (I) having less than 150 ppm (II). A low amount of (II) is relevant in view of regulatory requirements.
2) The method of the presently claimed invention is fast.
3) The method of the presently claimed invention is efficient as it provides a high yield of (I); in the range of 85 to 99 % based on the total of (I) in the feed.
4) The crystalline (I) obtained by the process of the presently claimed invention has a low amount of fines.

In the following, there is provided a list of embodiments to further illustrate the present disclosure without intending to limit the disclosure to the specific embodiments listed below.
1. A method for obtaining crystalline diethylamino hydroxybenzoyl hexyl benzoate (I) comprising steps (a) to (d),
   (a) providing a feed comprising diethylamino hydroxybenzoyl hexyl benzoate (I) and at least one crystallization solvent, and heating the feed to obtain a solution,
      wherein the diethylamino hydroxybenzoyl hexyl benzoate (I) has
         i) a phthalic acid dihexyl ester (PSDHE) (II) content in the range of 5000 to 30000 ppm;
         ii) a rhodamine (III) content in the range of 500 to 10000 ppm; and
         iii) a rhodamine hexyl ester (IV) content in the range of 500 to 10000 ppm; each based on the weight of diethylamino hydroxybenzoyl hexyl benzoate (I); and
      wherein the amount of the diethylamino hydroxybenzoyl hexyl benzoate (I) in the feed is in the range of 15.0 and 65.0 wt% based on the total weight of the feed,
   (b) cooling the solution of step (a) to a temperature such that the ratio of c : c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 to obtain a supersaturated solution of diethylamino hydroxybenzoyl hexyl benzoate (I);
   (c) seeding the supersaturated solution obtained in step (b) with seed crystals of diethylamino hydroxybenzoyl hexyl benzoate (I), followed by agitating the supersaturated solution comprising seed crystals for 1 to 8 hours;
      wherein the amount of the seed crystals is in the range from 0.1 to 15.0 wt% based on the weight of diethylamino hydroxybenzoyl hexyl benzoate (I) in the solution; and
   (d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
   (e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by the method steps (b) to (d).
2. A method for obtaining crystalline diethylamino hydroxybenzoyl hexyl benzoate (I) comprising steps (a) to (d),
   (a) providing a feed comprising diethylamino hydroxybenzoyl hexyl benzoate (I) and at least one crystallization solvent, and heating the feed to obtain a solution,
      wherein the diethylamino hydroxybenzoyl hexyl benzoate (I) has
         i) a phthalic acid dihexyl ester (PSDHE) (II) content in the range of 5000 to 30000 ppm;
         ii) a rhodamine (III) content in the range of 500 to 10000 ppm; and
         iii) a rhodamine hexyl ester (IV) content in the range of 500 to 10000 ppm; each based on the weight of diethylamino hydroxybenzoyl hexyl benzoate (I); and
      wherein the amount of the diethylamino hydroxybenzoyl hexyl benzoate (I) in the feed is in the range of 15.0 to 65.0 wt% based on the total weight of the feed;
   (b) cooling the solution obtained in step (a) to obtain a supersaturated solution of diethylamino hydroxybenzoyl hexyl benzoate (I);
   (c) seeding the supersaturated solution obtained in step (b) with seed crystals of diethylamino hydroxybenzoyl hexyl benzoate (I), followed by agitating the supersaturated solution comprising seed crystals for 1 to 8 hours;
      wherein the amount of the seed crystals is in the range from 0.1 to 15.0 wt% based on the weight of diethylamino hydroxybenzoyl hexyl benzoate (I) in the solution; and
   (d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
   (e) optionally recrystallizing the diethylamino hydroxybenzoyl hexyl benzoate (I) obtained in step (d) involving a step of mixing the diethylamino hydroxybenzoyl hexyl benzoate (I) obtained in step (d) with the at least one crystallization solvent used in step (a) to obtain a feed and heating the feed to obtain a solution, followed by the method steps (b) to (d).
3. The method according to embodiment 1 or 2, wherein the crystallization solvent is at least one selected from the group consisting of C₄-C₈ alcohols.
4. The method according to any of embodiments 1 to 3, wherein the crystallization solvent is 1-hexanol.
5. The method according to any of embodiments 1 to 4, wherein in step (a) the feed is heated to a temperature in the range of 25 to 80 °C.
6. The method according to any of claims 1 to 5, wherein in step (a) the feed is heated to a temperature in the range of 35 to 80 °C.
7. The method according to any of embodiments 1 to 6, wherein in step (b) the solution is cooled to a temperature in the range of by 20 to 35 °C.
8. The method according to any of embodiments 1 to 7, wherein in step (c) the seeding is carried out at a temperature in the range of 20 to 35 °C.
9. The method according to any of embodiments 1 to 8, wherein the seed crystals of diethylamino hydroxybenzoyl hexyl benzoate (I) are employed in step (c) in at least one form selected from a solid crystalline mass and a suspension in a solvent.
10. The method according to any of embodiments 1 to 9, wherein in step (d) the solution is cooled at a cooling rate of 1 to 15 °C/h.
11. The method according to any of embodiments 1 to 10, wherein in step (d) the solution is cooled in a linear manner.
12. The method according to any of embodiments 1 to 10, wherein in step (d) the solution is cooled with a piecewise linear cooling profile.
13. The method according to any of embodiments 1 to 10, wherein in step (d) the solution is cooled with a parabolic cooling profile with increasing cooling rates.
14. The method according to any of embodiments 1 to 10, wherein in step (d) the solution is cooled with a dynamic cooling profile to maintain a relatively constant supersaturation in the crystallizer.
15. The method according to any of embodiments 1 to 10, wherein in step (d) the solution is cooled with vacuum cooling method.
16. The method according to any of embodiments 1 to 15, wherein in step (d) the separation of crystals is carried out by filtration while maintaining the temperature of the solution within ± 5 °C of the temperature to which the solution is cooled in step (d).
17. The method according to any of embodiments 1 to 16, wherein step (d) further comprises a step of washing the separated crystalline diethylamino hydroxybenzoyl hexyl benzoate (I) with a washing solvent.
18. The method according to embodiment 17, wherein the washing of the separated crystalline diethylamino hydroxybenzoyl hexyl benzoate (I) is carried with washing solvent having a temperature within ± 5 °C of the temperature to which the solution is cooled in step (d).
19. The method according to embodiment 17 or 18, wherein the washing solvent is at least one selected from the crystallization solvent used in step (a), the feed and a saturated solution of (I) in the crystallization solvent used in step (a).
20. The method according to any of embodiments 1 to 19, further comprising the following steps,
   i. dissolving the crystalline diethylamino hydroxybenzoyl hexyl benzoate (I) obtained in step (d) or step (e) in a purification solvent selected from toluene and cyclohexane;
   ii. contacting the solution obtained in step (i) with an adsorbent selected from silica and charcoal; and
   iii. removal of solvent from the solution obtained in step (ii) to obtain crystalline diethylamino hydroxybenzoyl hexyl benzoate (I).
21. The method according to any of embodiments 1 to 20, wherein the crystallization is carried out in a crystallizer selected from a stirred vessel with baffles, force circulation crystallizer, draft tube crystallizer, draft tube baffled crystallizer, and an Oslo type crystallizer.
22. The method according to any of embodiments 1 to 21, wherein the crystalline diethylamino hydroxybenzoyl hexyl benzoate (I) has
   i) a phthalic acid dihexyl ester (PSDHE) (III) content in the range of 1 to 250 ppm;
   ii) a rhodamine (III) content in the range of 1 to 100 ppm; and
   iii) a rhodamine hexyl ester (IV) content in the range of 1 to 120 ppm;
   each based on the weight of the crystalline diethylamino hydroxybenzoyl hexyl benzoate (I).
23. Crystalline diethylamino hydroxybenzoyl hexyl benzoate (I) obtained by the method according to any of claims 1 to 22 having a phthalic acid dihexyl ester (PSDHE) (II) content in the range of 1 to 250 ppm, based on the weight of the crystalline diethylamino hydroxybenzoyl hexyl benzoate (I).
24. Crystalline diethylamino hydroxybenzoyl hexyl benzoate (I) obtained by the method according to any of embodiments 1 to 22 having
   i) a phthalic acid dihexyl ester (PSDHE) (II) content in the range of 1 to 250 ppm;
   ii) a rhodamine (III) content in the range of 1 to 100 ppm; and
   iii) a rhodamine hexyl ester (IV) content in the range of 1 to 120 ppm;
   each based on the weight of the crystalline diethylamino hydroxybenzoyl hexyl benzoate (I).
25. A cosmetic composition comprising crystalline diethylamino hydroxybenzoyl hexyl benzoate (I) and a carrier, wherein the crystalline diethylamino hydroxybenzoyl hexyl benzoate (I) has
   i) a phthalic acid dihexyl ester (PSDHE) (II) content in the range of 1 to 250 ppm;
   ii) a rhodamine (III) content in the range of 1 to 100 ppm; and
   iii) a rhodamine hexyl ester (IV) content in the range of 1 to 120 ppm,
   each based on the weight of crystalline diethylamino hydroxybenzoyl hexyl benzoate (I).

While the presently claimed invention has been described in terms of its specific embodiments, certain modifications and equivalents will be apparent to those skilled in the art and are intended to be included within the scope of the presently claimed invention.

### Examples

The presently claimed invention is illustrated in detail by non-restrictive working examples which follow. More particularly, the test methods specified hereinafter are part of the general disclosure of the application and are not restricted to the specific working examples.

### General

2-(4'-Diethylamino-2'-hydroxybenzoyl) benzoic acid hexyl ester (I) in hexanol was prepared according to US20050165099, example 2.

The product obtained from the process was subjected to crystallization as described in the experiments.

### Methods

H PLC method for determination of 2-(4'-diethylamino-2'-hydroxybenzoyl) benzoic acid hexyl ester (I), phthalate esters (II), and rhodamine type compounds (III) and (IV) have been performed by standard state of the art methods.

GC method for determination of phthalate esters and solvent residues have been performed by standard state of the art methods.

Analytical standards for calibration have been performed by standard state of the art methods.

### Solubility curve for 2-(4'-diethylamino-2'-hydroxybenzoyl)benzoic acid hexyl ester (I) in 1-hexanol

The equilibrium solubility of (I) in 1-hexanol at different temperatures was determined using a double jacketed 750 mL crystallizer. A mixture of (I) and 1-hexanol in the crystallizer was heated to a target temperature and the equilibrium condition was allowed to establish at that temperature. Samples from the solid free mother liquor were drawn using a filter frit. The samples were analysed for (I) content.

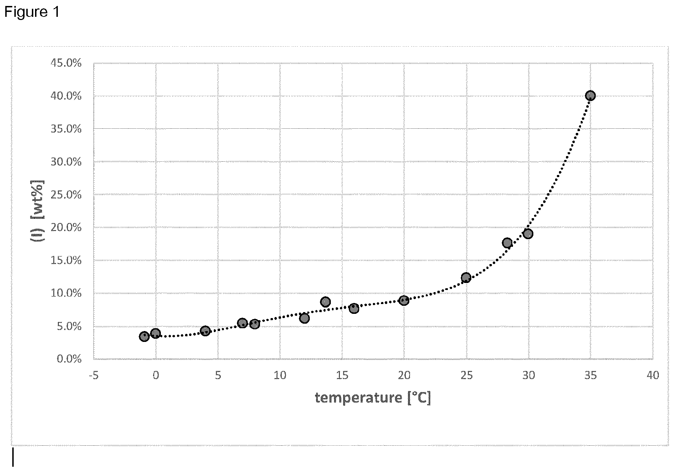

The results are shown in **Error! Reference source not found.**1 and are plotted in Figure 1.

**Table 1: Equilibrium solubility of (I) in 1-hexanol at different temperatures**

| **Temperature [°C]** | **(I) concentration** |
|---|---|
| -0.9 | 3.4% |
| 0 | 3.8% |
| 4 | 4.3% |
| 7 | 5.4% |
| 8 | 5.3% |
| 12 | 6.2% |
| 13.7 | 8.6% |
| 16 | 7.6% |
| 20 | 8.8% |
| 25 | 12.3% |
| 28.3 | 17.6% |
| 30 | 19.0% |
| 35 | 40.0% |

### Comparative examples according to WO 03097578

### Comparative Example C1: Crystallization according to WO 03097578

664.3 g of feed comprising 49.0 wt% 2-(4'-diethylamino-2'-hydroxybenzoyl)benzoic acid hexyl ester in hexanol containing 0.74 wt% di-n-hexylphthalate, 0.14 wt% rhodamine and 0.32 wt% rhodamine-hexyl ester corresponding to 14847 ppm di-n-hexylpthalate, 2786 ppm rhodamine and 6397 ppm rhodamine-hexylester calculated on 2-(4'-diethylamino-2'-hydroxybenzoyl)benzoic acid hexyl ester (s. above) was charged to a 1.6 L reactor.

The feed was heated at 40°C to obtain a solution. The solution was cooled to 20°C within 1.3 h to obtain a supersaturated solution having a c:c* ratio of 5.4.

After 4 h at 20°C crystallization started. The suspension was stirred at 20°C overnight (12 h), further cooled to 0°C within 4 h and stirred at 0°C. The product was filtered off and washed with a portion of 183 g pre-cooled (0°C) 1-hexanol and followed by another portion of 200 g pre-cooled (0°C) 1-hexanol to provide 371.5 g hexanol-wet 2-(4'-diethylamino-2'-hydroxybenzoyl)benzoic acid hexyl ester with a content of 80.6 wt% 2-(4'-diethylamino-2'-hydroxybenzoyl)benzoic acid hexyl ester.

Byproducts content calculated on the basis of (I): 110 ppm rhodamine, 131 ppm rhodamine-hexylester and 271 ppm di-n-hexylphthalate.

### Comparative Example C2: Crystallization according to WO 03097578

605.6 g of feed comprising 45.0 wt% 2-(4'-diethylamino-2'-hydroxybenzoyl)benzoic acid hexyl ester in hexanol containing 0.71 wt% di-n-hexylphthalate, 0.06 wt% rhodamine and 0.33 wt% rhodamine-hexylester corresponding to 15546 ppm di-n-hexylpthalate, 1298 ppm rhodamine and 7292 ppm rhodamine-hexylester calculated on 2-(4'-diethylamino-2'-hydroxybenzoyl)benzoic acid hexyl ester were charged to a 1.6 I reactor and the feed was heated at 40°C to obtain a solution.

The solution was cooled to 20°C within 1 h to obtain a supersaturated solution having a c:c* ratio of c:c* 5.0.

Crystallization started after 3.5 h at 20°C. The mixture was stirred for one more hour at 20°C followed by cooling to 0°C within 4 h and stirred at 0°C. The product was filtered off and washed with a first portion of 169 g pre-cooled (0°C) 1-hexanol followed by a second portion of 183 g pre-cooled (0°C) 1-hexanol to provide 310.9 g hexanol-wet 2-(4'-diethylamino-2'-hydroxybenzoyl)benzoic acid hexyl ester with a content of 79.1 wt% 2-(4'-diethylamino-2'-hydroxybenzoyl)benzoic acid hexyl ester.

Byproducts content calculated on the basis of (I): 73 ppm rhodamine, 187 ppm rhodamine-hexylester and 344 ppm di-n-hexylphthalate.

The process parameters and the results of comparative examples 1 and 2 are summarized in **Table 2.**

### Crystallization according to the present invention

### Example P1: Crystallization according to the invention using the same starting material as in comparative example 1

589.0 g of feed comprising 49.0 wt% 2-(4'-diethylamino-2'-hydroxybenzoyl)benzoic acid hexyl ester in hexanol containing 0.74 wt% di-n-hexylphthalate, 0.14 wt% rhodamine and 0.32 wt% rhodamine-hexylester corresponding to 14847 ppm di-n-hexylpthalate, 2786 ppm rhodamine and 6397 ppm rhodamine-hexylester calculated on 2-(4'-diethylamino-2'-hydroxybenzoyl)benzoic acid hexyl ester (s. above) was charged to a 1.6 L reactor and the feed was heated at 49°C to obtain a solution.

The solution was cooled to 25°C within 1.5 h to obtain a supersaturated solution having c:c* ratio of 4.1.

The supersaturated solution was seeded with 12.3 g (3.6 wt%) 2-(4'-diethylamino-2'-hydroxybenzoyl)benzoic acid hexyl ester (84.3 wt%) followed by stirring for 3.4 h at 25°C. The suspension was further cooled to 0°C within 5 h and stirred at 0°C. The product was filtered off and washed with a first portion of 162 g pre-cooled 1-hexanol (0°C) followed by a second portion of 177 g pre-cooled 1-hexanol (0°C) to provide 338 g hexanol-wet 2-(4'-diethylamino-2'-hydroxybenzoyl)benzoic acid hexyl ester (I) with a content of 80.2 wt% 2-(4'-diethylamino-2'-hydroxybenzoyl)benzoic acid hexyl ester (I).

Byproducts content calculated on basis of (I): 60 ppm rhodamine, 67 ppm rhodamine-hexylester and 68 ppm di-n-hexylphthalate.

### Example P2: Crystallization according to the invention using the same starting material as in comparative example 2

601.8 g of feed comprising 45.0 wt% 2-(4'-diethylamino-2'-hydroxybenzoyl)benzoic acid hexyl ester in hexanol containing 0.71 wt% di-n-hexylphthalate, 0.06 wt% rhodamine and 0.33 wt% rhodamine-hexylester corresponding to 15546 ppm di-n-hexylpthalate, 1298 ppm rhodamine and 7292 ppm rhodamine-hexylester calculated on 2-(4'-diethylamino-2'-hydroxybenzoyl)benzoic acid hexyl ester (s. above) was charged to a 1.6 L reactor and the feed was heated at 60°C to obtain a solution.

The solution was cooled to 25°C within 1 h to obtain a supersaturated solution having c:c* ratio of 3.8.

The supersaturated solution was seeded with 11 g (3.5 wt%) hexanol wet 2-(4'-diethylamino-2'-hydroxybenzoyl)benzoic acid hexyl ester (86.7 wt%) followed by stirring for 3.5 h at 25°C. The solution was cooled to 0°C within 5 h and stirred at 0°C. The product was filtered off and washed with a first portion of 168 g pre-cooled (0°C) 1-hexanol followed by a second portion of 182 g pre-cooled (0°C) hexanol to provide 306.4 g hexanol-wet 2-(4'-diethylamino-2'-hydroxybenzoyl)benzoic acid hexyl ester with a content of 82.1 wt% 2-(4'-diethylamino-2'-hydroxybenzoyl)benzoic acid hexyl ester.

Byproducts content calculated on (I): 30 ppm rhodamine, 105 ppm rhodamine-hexylester and 82 ppm di-n-hexylphthalate.

It is clear that the amount of impurities (II), (III) and (IV) in crystalline (I) obtained by the process of the presently claimed invention is low as compared to that obtained by the comparative examples.

### Examples P3-P11

The method used for crystallization of Examples P3-P11 was similar to method used for the crystallization for Example P1 according to of the presently claimed invention.

The process parameters and the results of examples P1 to P11 are summarized in **Table 3.**

### Recrystallization

### Example R1: Recrystallization of crystalline (I) obtained from C1

180.0 g of the hexanol wet filter cake obtained from C1 comprising 2-(4'-diethylamino-2'-hydroxybenzoyl)benzoic acid hexyl ester (80.6 wt%) and 120 g hexanol (48.4 wt% 2-(4'-diethylamino-2'-hydroxybenzoyl)benzoic acid hexyl ester in hexanol) was charged to a 1.6 I reactor and the feed was heated at 40°C to obtain a solution.

The solution was cooled to 25°C within 1.5 h to obtain a supersaturated solution having c:c* ratio of 4.1.

The supersaturated solution was seeded with 6.3 g (3.7 wt%) 2-(4'-diethylamino-2'-hydroxybenzoyl)benzoic acid hexyl ester (84.3 wt%) followed by stirring for 3.6 h at 25°C. The suspension was further cooled to 0°C within 5 h and stirred at 0°C. The suspension was filtered and the product washed with a first portion of 81 g pre-cooled (0°C) 1-hexanol and 89 g pre-cooled (0°C) 1-hexanol to provide 166.7 g hexanol-wet 2-(4'-diethylamino-2'-hydroxybenzoyl)benzoic acid hexyl ester with a content of 79.0 wt% 2-(4'-diethylamino-2'-hydroxybenzoyl)benzoic acid hexyl ester.

Byproducts content cal. on (I): 14 ppm rhodamine, 5 ppm rhodamine-hexylester and 12 ppm din-hexylphthalate.

### Example R2: Recrystallization

201.5 g of hexanol wet feed comprising 2-(4'-diethylamino-2'-hydroxybenzoyl)benzoic acid hexyl ester (88.9 wt%) containing 44 ppm rhodamine, 121 ppm rhodamine-hexylester and 165 ppm din-hexylphthalate calculated on 2-(4'-diethylamino-2'-hydroxybenzoyl)benzoic acid hexyl ester (s. above) and 172 g hexanol (48.0 wt% 2-(4'-diethylamino-2'-hydroxybenzoyl)benzoic acid hexyl ester in hexanol) was charged to a 1.6 I reactor and the feed was heated to 49°C to obtain a solution.

The solution was cooled to 25°C within 1.5 h to obtain a supersaturated solution having c:c* ratio of 4.0.

The supersaturated solution was seeded with 6.8 g (3.2 wt%) 2-(4'-diethylamino-2'-hydroxybenzoyl)benzoic acid hexyl ester (84.3 wt%) followed by stirring for 3.6 h at 25°C. The suspension was further cooled to 0°C within 5 h and stirred at 0°C. The suspension was filtered and the product washed with a first portion of 105 g pre-cooled (0°C) 1-hexanol and a second portion of 115 g pre-cooled (0°C) 1-hexanol to provide 187 g hexanol-wet 2-(4'-diethylamino-2'-hydroxybenzoyl)benzoic acid hexyl ester with a content of 82.5 wt% 2-(4'-diethylamino-2'-hydroxybenzoyl)benzoic acid hexyl ester.

Byproducts content calculated on the basis of 100% (I): 4 ppm rhodamine, 4 ppm rhodamine-hexylester and 4.5 ppm di-n-hexylphthalate.

### Examples R3 and R4

The method used for crystallization of Examples R3 and R4 was similar to method used for the crystallization for Example R2 according to of the presently claimed invention.

The process parameters and the results of examples R1 to R4 are summarized in **Table 4.**

**Table 2: Comparative examples**

| **Sr.** | **Feed** | | | | **Feed heating temp. - Step (a)** | **Solution cooling temp. - Step (b)** | **c:c*** | **Crystallization starting time** | **Stirring Time after crystallization start** | **Time to cool to 0 °C** | **Product** | | | |
|---|---|---|---|---|---|---|---|---|---|---|---|---|---|---|
| | Conc. Of (I) | II | III | IV | °C | °C | | h | h | h | Yield (%) | II (ppm) | III (ppm) | IV (ppm) |
| **C1** | 49.0 | 14847 | 2786 | 6397 | 40 | 20 | 5.4 | 4 | 12 | 4.0 | 90 | 271 | 110 | 131 |
| **C2** | 45.0 | 15546 | 1298 | 7292 | 40 | 20 | 5.0 | 3.5 | 1.0 | 4.0 | 90 | 344 | 73 | 187 |

**Table 3: Examples of the presently claimed invention**

| **Sr.** | **Feed** | | | | **Feed heating temp. - Step (a)** | **Solution cooling temp. - Step (b)** | **c:c*** | **Seeding amount** | **Stirring time after seeding** | **Time to cool to 0 °C** | **Product** | | | |
|---|---|---|---|---|---|---|---|---|---|---|---|---|---|---|
| | Conc. Of (I) | II | III | IV | °C | °C | | | h | h | Yield (%) | II (ppm) | III (ppm) | IV (ppm) |
| **P1** | 49.0 | 14847 | 2786 | 6397 | 49 | 25 | 4.1 | 3.6 | 3.6 | 5.0 | 94 | 68 | 60 | 67 |
| **P2** | 45.0 | 15546 | 1298 | 7292 | 60 | 25 | 3.8 | 3.5 | 3.5 | 5.0 | 93 | 82 | 30 | 105 |
| **P3** | 47.8 | 16641 | 1358 | 9818 | 47 | 25 | 4.0 | 1.5 | 2.5 | 5.7 | 90 | 66 | 30 | 89 |
| **P4** | 49.4 | 16910 | 1213 | 9779 | 44 | 25 | 4.2 | 1.5 | 3.6 | 5.5 | 90 | 59 | 26 | 98 |
| **P5** | 49.5 | 16921 | 1332 | 9841 | 47 | 29 | 2.8 | 1.5 | 2.5 | 5.7 | 89 | 49 | 28 | 113 |
| **P6** | 52.3 | 15546 | 1298 | 7292 | 60 | 26 | 4.0 | 3.5 | 3.6 | 5.0 | 86 | 62 | 25 | 80 |
| **P7** | 48.0 | 15709 | 1292 | 5524 | 60 | 26 | 3.7 | 1.5 | 3.6 | 5.0 | 85 | 38 | 28 | 65 |
| **P8** | 53.7 | 11822 | 2157 | 3362 | 48 | 25 | 4.5 | 4.0 | 3.6 | 5.0 | 92 | 147 | 81 | 81 |
| **P9** | 50.1 | 15550 | 1316 | 7905 | 45 | 25 | 4.2 | 3.5 | 3.6 | 5.0 | 86 | 59 | 27 | 71 |
| **P10** | 46.1 | 9386 | 710 | 692 | 46 | 25 | 3.9 | 1.6 | 3.6 | 5.5 | 90 | 39 | 13 | 23 |
| **P11** | 49.9 | 8412 | 581 | 539 | 58 | 25 | 4.2 | 3.6 | 3.6 | 5.0 | 92 | 69 | 14 | 17 |

**Table 4: Recrystallization**

| **Sr.** | **Feed** | | | | **Feed heating temp. - Step (a)** | **Solution coolIng temp. - Step (b)** | **c:c*** | **Seeding amount** | **Stirring time after seeding** | **Time to cool to 0 °C** | **Product** | | | |
|---|---|---|---|---|---|---|---|---|---|---|---|---|---|---|
| | Conc. Of (I) | II | III | IV | °C | °C | | | h | h | Yield (%) | II (ppm) | III (ppm) | IV (ppm) |
| **R1** | 48.4 | 271 | 93 | 59 | 40 | 25 | 4.1 | 3.7 | 3.6 | 5.0 | 91 | 12 | 14 | 5 |
| **R2** | 48.0 | 165 | 44 | 121 | 49 | 25 | 4.0 | 3.2 | 3.6 | 5.0 | 86 | 4.7 | 4 | 4 |
| **R3** | 48.0 | 83 | 22 | 84 | 58 | 25 | 4.0 | 1.5 | 3.6 | 5.0 | 86 | 1.5 | 2 | 2 |
| **R4** | 48.0 | 59 | 24 | 63 | 58 | 25 | 4.0 | 1.5 | 3.6 | 5.0 | 90 | 1.5 | 2 | 2 |

## Claims

1. A method for obtaining crystalline diethylamino hydroxybenzoyl hexyl benzoate (I) comprising steps (a) to (d),
(a) providing a feed comprising diethylamino hydroxybenzoyl hexyl benzoate (I) and at least one crystallization solvent, and heating the feed to obtain a solution,
wherein the diethylamino hydroxybenzoyl hexyl benzoate (I) has
i) a phthalic acid dihexyl ester (PSDHE) (II) content in the range of 5000 to 30000 ppm;
ii) a rhodamine (III) content in the range of 500 to 10000 ppm; and
iii) a rhodamine hexyl ester (IV) content in the range of 500 to 10000 ppm; each based on the weight of diethylamino hydroxybenzoyl hexyl benzoate (I); and
wherein the amount of the diethylamino hydroxybenzoyl hexyl benzoate (I) in the feed is in the range of 15.0 and 65.0 wt% based on the total weight of the feed,
(b) cooling the solution of step (a) to a temperature such that the ratio of c : c* of the concentration c of dissolved diethylamino hydroxybenzoyl hexyl benzoate (I) to the equilibrium solubility c* of diethylamino hydroxybenzoyl hexyl benzoate (I) at the temperature is in the range from 1.1:1.0 to 9.0:1.0 to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with the at least one crystallization solvent as used in step (a) to obtain a feed, heating the feed to obtain a solution, and using the method steps (b) to (d).

2. A method for obtaining crystalline diethylamino hydroxybenzoyl hexyl benzoate (I) comprising steps (a) to (d),
(a) providing a feed comprising diethylamino hydroxybenzoyl hexyl benzoate (I) and at least one crystallization solvent, and heating the feed to obtain a solution,
wherein the diethylamino hydroxybenzoyl hexyl benzoate (I) has
i) a phthalic acid dihexyl ester (PSDHE) (II) content in the range of 5000 to 30000 ppm;
ii) a rhodamine (III) content in the range of 500 to 10000 ppm; and
iii) a rhodamine hexyl ester (IV) content in the range of 500 to 10000 ppm; each based on the weight of diethylamino hydroxybenzoyl hexyl benzoate (I); and
wherein the amount of the diethylamino hydroxybenzoyl hexyl benzoate (I) in the feed is in the range of 15.0 to 65.0 wt% based on the total weight of the feed;
(b) cooling the solution obtained in step (a) to obtain a supersaturated solution of diethylamino hydroxybenzoyl hexyl benzoate (I);
(c) seeding the supersaturated solution obtained in step (b) with seed crystals of diethylamino hydroxybenzoyl hexyl benzoate (I), followed by agitating the supersaturated solution comprising seed crystals for 1 to 8 hours;
wherein the amount of the seed crystals is in the range from 0.1 to 15.0 wt% based on the weight of diethylamino hydroxybenzoyl hexyl benzoate (I) in the solution; and
(d) cooling the solution obtained in step (c) to a temperature in the range of -10 to 15 °C to obtain a solution comprising crystalline diethylamino hydroxybenzoyl hexyl benzoate (I); followed by separating crystalline diethylamino hydroxybenzoyl hexyl benzoate (I); and
(e) optionally recrystallizing the diethylamino hydroxybenzoyl hexyl benzoate (I) obtained in step (d) involving mixing the diethylamino hydroxybenzoyl hexyl benzoate (I) obtained in step (d) with the at least one crystallization solvent as used in step (a) to obtain a feed, heating the feed to obtain a solution, and using the method steps (b) to (d).

3. The method according to claim 1 or 2, wherein the crystallization solvent is at least one selected from the group consisting of C₄-C₈ alcohols.

4. The method according to any of claims 1 to 3, wherein the crystallization solvent is 1-hexanol.

5. The method according to any of claims 1 to 4, wherein in step (a) the feed is heated to a temperature in the range of 25 to 80 °C.

6. The method according to any of claims 1 to 5, wherein in step (a) the feed is heated to a temperature in the range of 35 to 80 °C.

7. The method according to any of claims 1 to 6, wherein in step (b) the solution is cooled to a temperature in the range of by 20 to 35 °C.

8. The method according to any of claims 1 to 7, wherein in step (c) the seeding is carried out at a temperature in the range of 20 to 35 °C.

9. The method according to any of claims 1 to 8, wherein the seed crystals of diethylamino hydroxybenzoyl hexyl benzoate (I) are employed in step (c) in at least one form selected from a solid crystalline mass and a suspension in a solvent.

10. The method according to any of claims 1 to 9, wherein in step (d) the solution is cooled at a cooling rate of 1 to 15 °C/h.

11. The method according to any of claims 1 to 10, wherein in step (d) the solution is cooled in a linear manner.

12. The method according to any of claims 1 to 10, wherein in step (d) the solution is cooled with a piecewise linear cooling profile.

13. The method according to any of claims 1 to 10, wherein in step (d) the solution is cooled with a parabolic cooling profile with increasing cooling rates.

14. The method according to any of claims 1 to 10, wherein in step (d) the solution is cooled with a dynamic cooling profile to maintain a relatively constant supersaturation in the crystallizer.

15. The method according to any of claims 1 to 10, wherein in step (d) the solution is cooled with vacuum cooling method.

16. The method according to any of claims 1 to 15, wherein in step (d) the separation of crystals is carried out by filtration while maintaining the temperature of the solution within ± 5 °C of the temperature to which the solution is cooled in step (d).

17. The method according to any of claims 1 to 16, wherein step (d) further comprises a step of washing the separated crystalline diethylamino hydroxybenzoyl hexyl benzoate (I) with a washing solvent.

18. The method according to claim 17, wherein the washing of the separated crystalline diethylamino hydroxybenzoyl hexyl benzoate (I) is carried with washing solvent having a temperature within ± 5 °C of the temperature to which the solution is cooled in step (d).

19. The method according to claim 17 or 18, wherein the washing solvent is at least one selected from the crystallization solvent used in step (a), the feed and a saturated solution of (I) in the crystallization solvent used in step (a).

20. The method according to any of claims 1 to 19, further comprising the following steps,
i. dissolving the crystalline diethylamino hydroxybenzoyl hexyl benzoate (I) obtained in step (d) or step (e) in a purification solvent selected from toluene and cyclohexane;
ii. contacting the solution obtained in step (i) with an adsorbent selected from silica and charcoal; and
iii. removal of solvent from the solution obtained in step (ii) to obtain crystalline diethylamino hydroxybenzoyl hexyl benzoate (I).

21. The method according to any of claims 1 to 20, wherein the crystallization is carried out in a crystallizer selected from a stirred vessel with baffles, force circulation crystallizer, draft tube crystallizer, draft tube baffled crystallizer, and an Oslo type crystallizer.

22. The method according to any of claims 1 to 21, wherein the crystalline diethylamino hydroxybenzoyl hexyl benzoate (I) has
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm;
each based on the weight of the crystalline diethylamino hydroxybenzoyl hexyl benzoate (I).

23. Crystalline diethylamino hydroxybenzoyl hexyl benzoate (I) obtained by the method according to any of claims 1 to 22 having a phthalic acid dihexyl ester (PSDHE) content in the range of 1 to 250 ppm, based on the weight of the crystalline diethylamino hydroxybenzoyl hexyl benzoate (I).

24. Crystalline diethylamino hydroxybenzoyl hexyl benzoate (I) obtained by the method according to any of claims 1 to 22 having
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm;
each based on the weight of the crystalline diethylamino hydroxybenzoyl hexyl benzoate (I).

25. A cosmetic composition comprising crystalline diethylamino hydroxybenzoyl hexyl benzoate (I) and a carrier, wherein the crystalline diethylamino hydroxybenzoyl hexyl benzoate (I) has
i) a phthalic acid dihexyl ester (PSDHE) (II) content in the range of 1 to 250 ppm;
ii) a rhodamine (III) content in the range of 1 to 100 ppm; and
iii) a rhodamine hexyl ester (IV) content in the range of 1 to 120 ppm,
each based on the weight of crystalline diethylamino hydroxybenzoyl hexyl benzoate (I).

## Patentansprüche

1. Verfahren zum Herstellen von kristallinem Diethylaminohydroxybenzoylhexylbenzoat (I), umfassend die Schritte (a) bis (d),
(a) Bereitstellen einer Zufuhr, die Diethylaminohydroxybenzoylhexylbenzoat (I) und mindestens ein Kristallisationslösemittel umfasst, und Erwärmen der Zufuhr, um eine Lösung zu erhalten, wobei das Diethylaminohydroxybenzoylhexylbenzoat (I) Folgendes aufweist:
i) einen Gehalt an Phthalsäuredihexylester (PSDHE) (II) im Bereich von 5000 bis 30000 ppm;
ii) einen Gehalt an Rhodamin (III) im Bereich von 500 bis 10000 ppm; und
iii) einen Gehalt an Rhodaminhexylester (IV) im Bereich von 500 bis 10000 ppm; jeweils bezogen auf das Gewicht an Diethylaminohydroxybenzoylhexylbenzoat (I); und
wobei die Menge des Diethylaminohydroxybenzoylhexylbenzoats (I) in der Zufuhr im Bereich von 15,0 und 65,0 Gew.-%, bezogen auf das Gesamtgewicht der Zufuhr, liegt,
(b) Abkühlen der Lösung von Schritt (a) auf eine Temperatur, sodass das Verhältnis von c : c* der Konzentration c von gelöstem Diethylaminohydroxybenzoylhexylbenzoat (I) zu der Gleichgewichtslöslichkeit c* von Diethylaminohydroxybenzoylhexylbenzoat (I) bei der Temperatur im Bereich von 1,1 : 1,0 bis 9,0 : 1,0 liegt, um eine übersättigte Lösung von Diethylaminohydroxybenzoylhexylbenzoat (I) zu erhalten;
(c) Beimpfen der in Schritt (b) erhaltenen übersättigten Lösung mit Impfkristallen von Diethylaminohydroxybenzoylhexylbenzoat (I), gefolgt von einem Bewegen der übersättigten Lösung, die Impfkristalle umfasst, für 1 bis 8 Stunden;
wobei die Menge der Impfkristalle im Bereich von 0,1 bis 15,0 Gew.-%, bezogen auf das Gewicht von Diethylaminohydroxybenzoylhexylbenzoat (I) in der Lösung, liegt; und
(d) Abkühlen der in Schritt (c) erhaltenen Lösung auf eine Temperatur im Bereich von -10 bis 15 °C, um eine Lösung zu erhalten, die kristallines Diethylaminohydroxybenzoylhexylbenzoat (I) umfasst; gefolgt von einem Abtrennen des kristallinen Diethylaminohydroxybenzoylhexylbenzoats (I); und
(e) gegebenenfalls Umkristallisieren des in Schritt (d) erhaltenen Diethylaminohydroxybenzoylhexylbenzoats (I), das das Mischen des in Schritt (d) erhaltenen Diethylaminohydroxybenzoylhexylbenzoats (I) mit dem mindestens einen Kristallisationslösemittel, wie in Schritt (a) verwendet, umfasst, um eine Zufuhr zu erhalten, Erwärmen der Zufuhr, um eine Lösung zu erhalten, und Verwenden der Verfahrensschritte (b) bis (d).

2. Verfahren zum Herstellen von kristallinem Diethylaminohydroxybenzoylhexylbenzoat (I), umfassend die Schritte (a) bis (d),
(a) Bereitstellen einer Zufuhr, die Diethylaminohydroxybenzoylhexylbenzoat (I) und mindestens ein Kristallisationslösemittel umfasst, und Erwärmen der Zufuhr, um eine Lösung zu erhalten, wobei das Diethylaminohydroxybenzoylhexylbenzoat (I) Folgendes aufweist:
i) einen Gehalt an Phthalsäuredihexylester (PSDHE) (II) im Bereich von 5000 bis 30000 ppm;
ii) einen Gehalt an Rhodamin (III) im Bereich von 500 bis 10000 ppm; und
iii) einen Gehalt an Rhodaminhexylester (IV) im Bereich von 500 bis 10000 ppm; jeweils bezogen auf das Gewicht an Diethylaminohydroxybenzoylhexylbenzoat (I); und
wobei die Menge des Diethylaminohydroxybenzoylhexylbenzoats (I) in der Zufuhr im Bereich von 15,0 bis 65,0 Gew.-%, bezogen auf das Gesamtgewicht der Zufuhr, liegt,
(b) Abkühlen der in Schritt (a) erhaltenen Lösung, um eine übersättigte Lösung von Diethylaminohydroxybenzoylhexylbenzoat (I) zu erhalten;
(c) Beimpfen der in Schritt (b) erhaltenen übersättigten Lösung mit Impfkristallen von Diethylaminohydroxybenzoylhexylbenzoat (I), gefolgt von einem Bewegen der übersättigten Lösung, die Impfkristalle umfasst, für 1 bis 8 Stunden;
wobei die Menge der Impfkristalle im Bereich von 0,1 bis 15,0 Gew.-%, bezogen auf das Gewicht von Diethylaminohydroxybenzoylhexylbenzoat (I) in der Lösung, liegt; und
(d) Abkühlen der in Schritt (c) erhaltenen Lösung auf eine Temperatur im Bereich von -10 bis 15 °C, um eine Lösung zu erhalten, die kristallines Diethylaminohydroxybenzoylhexylbenzoat (I) umfasst; gefolgt von einem Abtrennen des kristallinen Diethylaminohydroxybenzoylhexylbenzoats (I); und
(e) gegebenenfalls Umkristallisieren des in Schritt (d) erhaltenen Diethylaminohydroxybenzoylhexylbenzoats (I), das das Mischen des in Schritt (d) erhaltenen Diethylaminohydroxybenzoylhexylbenzoats (I) mit dem mindestens einen Kristallisationslösemittel, wie in Schritt (a) verwendet, umfasst, um eine Zufuhr zu erhalten, Erwärmen der Zufuhr, um eine Lösung zu erhalten, und Verwenden der Verfahrensschritte (b) bis (d).

3. Verfahren nach Anspruch 1 oder 2, wobei das Kristallisationslösemittel mindestens eines ist, das ausgewählt ist aus der Gruppe bestehend aus C₄-C₈-Alkoholen.

4. Verfahren nach einem der Ansprüche 1 bis 3, wobei das Kristallisationslösemittel 1-Hexanol ist.

5. Verfahren nach einem der Ansprüche 1 bis 4, wobei in Schritt a) die Zufuhr auf eine Temperatur im Bereich von 25 °C bis 80 °C erwärmt wird.

6. Verfahren nach einem der Ansprüche 1 bis 5, wobei in Schritt a) die Zufuhr auf eine Temperatur im Bereich von 35 °C bis 80 °C erwärmt wird.

7. Verfahren nach einem der Ansprüche 1 bis 6, wobei in Schritt (b) die Lösung auf eine Temperatur im Bereich von 20 bis 35 °C abgekühlt wird.

8. Verfahren nach einem der Ansprüche 1 bis 7, wobei in Schritt (c) das Beimpfen bei einer Temperatur im Bereich von 20 °C bis 35 °C durchgeführt wird.

9. Verfahren nach einem der Ansprüche 1 bis 8, wobei die Impfkristalle von Diethylaminohydroxybenzoylhexylbenzoat (I) in Schritt (c) in mindestens einer Form eingesetzt werden, die ausgewählt ist aus einer festen kristallinen Masse und einer Suspension in einem Lösemittel.

10. Verfahren nach einem der Ansprüche 1 bis 9, wobei in Schritt (d) die Lösung mit einer Kühlrate von 1 bis 15 °C/h abgekühlt wird.

11. Verfahren nach einem der Ansprüche 1 bis 10, wobei in Schritt (d) die Lösung auf lineare Weise abgekühlt wird.

12. Verfahren nach einem der Ansprüche 1 bis 10, wobei in Schritt (d) die Lösung mit einem stückweisen linearen Kühlprofil abgekühlt wird.

13. Verfahren nach einem der Ansprüche 1 bis 10, wobei in Schritt (d) die Lösung mit einem parabolischen Kühlprofil mit steigenden Kühlraten abgekühlt wird.

14. Verfahren nach einem der Ansprüche 1 bis 10, wobei in Schritt (d) die Lösung mit einem dynamischen Kühlprofil abgekühlt wird, um eine relativ konstante Übersättigung in dem Kristallisator aufrechtzuerhalten.

15. Verfahren nach einem der Ansprüche 1 bis 10, wobei in Schritt (d) die Lösung mit einem Vakuumkühlverfahren abgekühlt wird.

16. Verfahren nach einem der Ansprüche 1 bis 15, wobei in Schritt (d) die Abtrennung der Kristalle durch Filtration durchgeführt wird, während die Temperatur der Lösung innerhalb von ± 5 °C der Temperatur gehalten wird, auf die die Lösung in Schritt (d) abgekühlt wird.

17. Verfahren nach einem der Ansprüche 1 bis 16, wobei Schritt (d) ferner einen Schritt des Waschens des abgetrennten kristallinen Diethylaminohydroxybenzoylhexylbenzoats (I) mit einem Waschlösemittel umfasst.

18. Verfahren nach Anspruch 17, wobei das Waschen des abgetrennten kristallinen Diethylaminohydroxybenzoylhexylbenzoats (I) mit Waschlösemittel mit einer Temperatur innerhalb von ± 5 °C der Temperatur durchgeführt wird, auf die die Lösung in Schritt (d) abgekühlt wird.

19. Verfahren nach Anspruch 17 oder 18, wobei das Waschlösemittel mindestens eines ist, das ausgewählt ist aus Kristallisationslösemittel, das in Schritt (a) verwendet wird, der Zufuhr und einer gesättigten Lösung von (I) in dem Kristallisationslösemittel, das in Schritt (a) verwendet wird.

20. Verfahren nach einem der Ansprüche 1 bis 19, ferner umfassend die folgenden Schritte:
i. Auflösen des in Schritt (d) oder Schritt (e) erhaltenen kristallinen Diethylaminohydroxybenzoylhexylbenzoats (I) in einem Reinigungslösemittel, das ausgewählt ist aus Toluol und Cyclohexan;
ii. Inkontaktbringen der in Schritt (i) erhaltenen Lösung mit einem Adsorptionsmittel, das ausgewählt ist aus Siliciumdioxid und Kohle; und
iii. Entfernung von Lösemittel aus der in Schritt (ii) erhaltenen Lösung, um kristallines Diethylaminohydroxybenzoylhexylbenzoat (I) zu erhalten.

21. Verfahren nach einem der Ansprüche 1 bis 20, wobei die Kristallisation in einem Kristallisator durchgeführt wird, der ausgewählt ist aus einem gerührten Gefäß mit Stromstörern, Zwangsumlaufkristallisator, Leitrohrkristallisator, Leitrohrkristallisator mit Feinkornauflösung und einem Oslo-Kristallisator.

22. Verfahren nach einem der Ansprüche 1 bis 21, wobei das kristalline Diethylaminohydroxybenzoylhexylbenzoat (I) Folgendes aufweist:
i) einen Gehalt an Phthalsäuredihexylester (PSDHE) (II) im Bereich von 1 bis 250 ppm;
ii) einen Gehalt an Rhodamin (III) im Bereich von 1 bis 100 ppm; und
iii) einen Gehalt an Rhodaminhexylester (IV) im Bereich von 1 bis 120 ppm;
jeweils bezogen auf das Gewicht des kristallinen Diethylaminohydroxybenzoylhexylbenzoats (I).

23. Kristallines Diethylaminohydroxybenzoylhexylbenzoat (I), erhalten durch das Verfahren nach einem der Ansprüche 1 bis 22, mit einem Gehalt an Phthalsäuredihexylester (PSDHE) im Bereich von 1 bis 250 ppm, bezogen auf das Gewicht des kristallinen Diethylaminohydroxybenzoylhexylbenzoats (I).

24. Kristallines Diethylaminohydroxybenzoylhexylbenzoat (I), erhalten durch das Verfahren nach einem der Ansprüche 1 bis 22, das Folgendes aufweist:
i) einen Gehalt an Phthalsäuredihexylester (PSDHE) (II) im Bereich von 1 bis 250 ppm;
ii) einen Gehalt an Rhodamin (III) im Bereich von 1 bis 100 ppm; und
iii) einen Gehalt an Rhodaminhexylester (IV) im Bereich von 1 bis 120 ppm;
jeweils bezogen auf das Gewicht des kristallinen Diethylaminohydroxybenzoylhexylbenzoats (I).

25. Kosmetische Zusammensetzung, umfassend kristallines Diethylaminohydroxybenzoylhexylbenzoat (I) und einen Träger, wobei das kristalline Diethylaminohydroxybenzoylhexylbenzoat (I) Folgendes aufweist:
i) einen Gehalt an Phthalsäuredihexylester (PSDHE) (II) im Bereich von 1 bis 250 ppm;
ii) einen Gehalt an Rhodamin (III) im Bereich von 1 bis 100 ppm; und
iii) einen Gehalt an Rhodaminhexylester (IV) im Bereich von 1 bis 120 ppm; jeweils bezogen auf das Gewicht an Diethylaminohydroxybenzoylhexylbenzoat (I).

## Revendications

1. Procédé d'obtention de benzoate de diéthylaminohydroxybenzoylhexyle cristallin (I) comprenant les étapes (a) à (d),
(a) fourniture d'une charge comprenant du benzoate de diéthylaminohydroxybenzoylhexyle (I) et d'au moins un solvant de cristallisation, et chauffage de la charge pour obtenir une solution, dans lequel le benzoate de diéthylaminohydroxybenzoylhexyle (I) a
i) une teneur en ester dihexylique de l'acide phtalique (PSDHE) (II) dans la plage de 5 000 à 30 000 ppm ;
ii) une teneur en rhodamine (III) dans la plage de 500 à 10 000 ppm ; et
iii) une teneur en ester hexylique de rhodamine (IV) dans la plage de 500 à 10 000 ppm ; chacune basée sur le poids du benzoate de diéthylaminohydroxybenzoylhexyle (I) ; et dans lequel la quantité de benzoate de diéthylaminohydroxybenzoylhexyle (I) dans la charge est dans la plage de 15,0 à 65,0 % en poids par rapport au poids total de la charge,
(b) refroidissement de la solution de l'étape (a) jusqu'à une température telle que le rapport c : c* de la concentration c de benzoate de diéthylaminohydroxybenzoylhexyle dissous (I) sur la solubilité à l'équilibre c* du benzoate de diéthylaminohydroxybenzoylhexyle (I) à la température soit dans la plage de 1,1 : 1,0 à 9,0 : 1,0, pour obtenir une solution sursaturée de benzoate de diéthylaminohydroxybenzoylhexyle (I) ;
c) ensemencement de la solution sursaturée obtenue à l'étape (b) avec des germes cristallins de benzoate de diéthylaminohydroxybenzoylhexyle (I), suivi d'une agitation de la solution sursaturée comprenant des germes cristallins pendant 1 à 8 heures ;
dans lequel la quantité des germes cristallins est dans la plage de 0,1 à 15,0 % en poids par rapport au poids du benzoate de diéthylaminohydroxybenzoylhexyle (I) dans la solution ; et
(d) refroidissement de la solution obtenue à l'étape (c) jusqu'à une température dans la plage de -10 à 15 °C pour obtenir une solution comprenant du benzoate de diéthylaminohydroxybenzoylhexyle cristallin (I) ; puis séparation du benzoate de diéthylaminohydroxybenzoylhexyle cristallin (I) ; et
(e) éventuellement recristallisation du benzoate de diéthylaminohydroxybenzoylhexyle (I) obtenu à l'étape (d) en mélangeant le benzoate de diéthylaminohydroxybenzoylhexyle (I) obtenu à l'étape (d) avec l'au moins un solvant de cristallisation utilisé à l'étape (a) pour obtenir une charge, chauffage de la charge pour obtenir une solution, et utilisation des étapes (b) à (d) du procédé.

2. Procédé d'obtention de benzoate de diéthylaminohydroxybenzoylhexyle cristallin (I) comprenant les étapes (a) à (d),
(a) fourniture d'une charge comprenant du benzoate de diéthylaminohydroxybenzoylhexyle (I) et d'au moins un solvant de cristallisation, et chauffage de la charge pour obtenir une solution, dans lequel le benzoate de diéthylaminohydroxybenzoylhexyle (I) a
i) une teneur en ester dihexylique de l'acide phtalique (PSDHE) (II) dans la plage de 5 000 à 30 000 ppm ;
ii) une teneur en rhodamine (III) dans la plage de 500 à 10 000 ppm ; et
iii) une teneur en ester hexylique de rhodamine (IV) dans la plage de 500 à 10 000 ppm ; chacune basée sur le poids du benzoate de diéthylaminohydroxybenzoylhexyle (I) ; et dans lequel la quantité de benzoate de diéthylaminohydroxybenzoylhexyle (I) dans la charge est dans la plage de 15,0 à 65,0 % en poids par rapport au poids total de la charge ;
(b) refroidissement de la solution obtenue à l'étape (a) pour obtenir une solution sursaturée de benzoate de diéthylaminohydroxybenzoylhexyle (I) ;
(c) ensemencement de la solution sursaturée obtenue à l'étape (b) avec des germes cristallins de benzoate de diéthylaminohydroxybenzoylhexyle (I), suivi d'une agitation de la solution sursaturée comprenant des germes cristallins pendant 1 à 8 heures ;
dans lequel la quantité des germes cristallins est dans la plage de 0,1 à 15,0 % en poids par rapport au poids du benzoate de diéthylaminohydroxybenzoylhexyle (I) dans la solution ; et
(d) refroidissement de la solution obtenue à l'étape (c) jusqu'à une température dans la plage de -10 à 15 °C pour obtenir une solution comprenant du benzoate de diéthylaminohydroxybenzoylhexyle cristallin (I) ; puis séparation du benzoate de diéthylaminohydroxybenzoylhexyle cristallin (I) ; et
(e) éventuellement recristallisation du benzoate de diéthylaminohydroxybenzoylhexyle (I) obtenu à l'étape (d) en mélangeant le benzoate de diéthylaminohydroxybenzoylhexyle (I) obtenu à l'étape (d) avec l'au moins un solvant de cristallisation utilisé à l'étape (a) pour obtenir une charge, chauffage de la charge pour obtenir une solution, et utilisation des étapes (b) à (d) du procédé.

3. Procédé selon la revendication 1 ou 2, dans lequel le solvant de cristallisation est au moins un solvant choisi dans le groupe constitué par les alcools en C₄-C₈.

4. Procédé selon l'une quelconque des revendications 1 à 3, dans lequel le solvant de cristallisation est le 1-hexanol.

5. Procédé selon l'une quelconque des revendications 1 à 4, dans lequel dans l'étape (a) la charge est chauffée jusqu'à une température dans la plage de 25 à 80 °C.

6. Procédé selon l'une quelconque des revendications 1 à 5, dans lequel dans l'étape (a) la charge est chauffée jusqu'à une température dans la plage de 35 à 80 °C.

7. Procédé selon l'une des revendications 1 à 6, dans lequel dans l'étape (b) la solution est refroidie jusqu'à une température dans la plage de 20 à 35 °C.

8. Procédé selon l'une quelconque des revendications 1 à 7, dans lequel dans l'étape (c) l'ensemencement est réalisé à une température dans la plage de 20 à 35 °C.

9. Procédé selon l'une quelconque des revendications 1 à 8, dans lequel les germes cristallins de benzoate de diéthylaminohydroxybenzoylhexyle (I) sont utilisés dans l'étape (c) sous au moins une forme choisie parmi une masse cristalline solide et une suspension dans un solvant.

10. Procédé selon l'une quelconque des revendications 1 à 9, dans lequel, dans l'étape (d), la solution est refroidie à une vitesse de refroidissement de 1 à 15 °C/h.

11. Procédé selon l'une quelconque des revendications 1 à 10, dans lequel, dans l'étape (d), la solution est refroidie d'une manière linéaire.

12. Procédé selon l'une quelconque des revendications 1 à 10, dans lequel, dans l'étape (d), la solution est refroidie avec un profil de refroidissement linéaire par morceaux.

13. Procédé selon l'une quelconque des revendications 1 à 10, dans lequel, dans l'étape (d), la solution est refroidie avec un profil de refroidissement parabolique avec des vitesses de refroidissement croissantes.

14. Procédé selon l'une quelconque des revendications 1 à 10, dans lequel, dans l'étape (d), la solution est refroidie avec un profil de refroidissement dynamique pour maintenir une sursaturation relativement constante dans le cristallisoir.

15. Procédé selon l'une quelconque des revendications 1 à 10, dans lequel, dans l'étape (d), la solution est refroidie par un procédé de refroidissement sous vide.

16. Procédé selon l'une quelconque des revendications 1 à 15, dans lequel, dans l'étape (d), la séparation de cristaux est effectuée par filtration tout en maintenant la température de la solution à ± 5 °C de la température à laquelle la solution est refroidie dans l'étape (d).

17. Procédé selon l'une quelconque des revendications 1 à 16, dans lequel l'étape (d) comprend en outre une étape de lavage du benzoate de diéthylaminohydroxybenzoylhexyle cristallin séparé (i) avec un solvant de lavage.

18. Procédé selon la revendication 17, dans lequel le lavage du benzoate de diéthylaminohydroxybenzoylhexyle cristallin séparé (I) est effectué avec un solvant de lavage ayant une température à ± 5 °C de la température à laquelle la solution est refroidie dans l'étape (d).

19. Procédé selon la revendication 17 ou 18, dans lequel le solvant de lavage est au moins un solvant choisi parmi le solvant de cristallisation utilisé dans l'étape (a), la charge et une solution saturée de (I) dans le solvant de cristallisation utilisé dans l'étape (a).

20. Procédé selon l'une quelconque des revendications 1 à 19, comprenant en outre les étapes suivantes,
i. dissolution du benzoate de diéthylaminohydroxybenzoylhexyle cristallin (I) obtenu à l'étape (d) ou à l'étape (e) dans un solvant de purification choisi parmi le toluène et le cyclohexane ;
ii. mise en contact de la solution obtenue à l'étape (i) avec un adsorbant choisi parmi la silice et le charbon ; et
iii. élimination de solvant de la solution obtenue à l'étape (ii) pour obtenir le benzoate de diéthylaminohydroxybenzoylhexyle cristallin (I).

21. Procédé selon l'une quelconque des revendications 1 à 20, dans lequel la cristallisation est effectuée dans un cristallisoir choisi parmi un récipient agité avec des chicanes, un cristallisoir à circulation forcée, un cristallisoir à tube d'aspiration, un cristallisoir à chicanes à tube d'aspiration et un cristallisoir de type Oslo.

22. Procédé selon l'une quelconque des revendications 1 à 21, dans lequel le benzoate de diéthylaminohydroxybenzoylhexyle cristallin (I) a
i) une teneur en ester dihexylique de l'acide phtalique (PSDHE) (II) dans la plage de 1 à 250 ppm ;
ii) une teneur en rhodamine (III) dans la plage de 1 à 100 ppm ; et
iii) une teneur en ester hexylique de rhodamine (IV) dans la plage de 1 à 120 ppm ;
chacune basée sur le poids du benzoate de diéthylaminohydroxybenzoylhexyle cristallin (I).

23. Benzoate de diéthylaminohydroxybenzoylhexyle cristallin (I) obtenu par le procédé selon l'une quelconque des revendications 1 à 22, ayant une teneur en ester dihexylique de l'acide phtalique (PSDHE) dans la plage de 1 à 250 ppm, par rapport au poids du benzoate de diéthylaminohydroxybenzoylhexyle cristallin (I).

24. Benzoate de diéthylaminohydroxybenzoylhexyle cristallin (I) obtenu par le procédé selon l'une quelconque des revendications 1 à 22 ayant
i) une teneur en ester dihexylique de l'acide phtalique (PSDHE) (II) dans la plage de 1 à 250 ppm ;
ii) une teneur en rhodamine (III) dans la plage de 1 à 100 ppm ; et
iii) une teneur en ester hexylique de rhodamine (IV) dans la plage de 1 à 120 ppm ;
chacune basée sur le poids du benzoate de diéthylaminohydroxybenzoylhexyle cristallin (I).

25. Composition cosmétique comprenant du benzoate de diéthylaminohydroxybenzoylhexyle cristallin (I) et un support, dans laquelle le benzoate de diéthylaminohydroxybenzoylhexyle cristallin (I) a
i) une teneur en ester dihexylique de l'acide phtalique (PSDHE) (II) dans la plage de 1 à 250 ppm ;
ii) une teneur en rhodamine (III) dans la plage de 1 à 100 ppm ; et
iii) une teneur en ester hexylique de rhodamine (IV) dans la plage de 1 à 120 ppm, chacune basée sur le poids du benzoate de diéthylaminohydroxybenzoylhexyle cristallin (I) .
